Europäisches Patentamt

European Patent Office

Office européen des brevets

(11) Veröffentlichungsnummer: **0 621 021 A1**

## (12) EUROPÄISCHE PATENTANMELDUNG

(21) Anmeldenummer: **93106409.1**

(22) Anmeldetag: **20.04.93**

(51) Int. Cl.5: **A61F 4/00**, H01H 3/14, G06F 3/00

(43) Veröffentlichungstag der Anmeldung:
**26.10.94 Patentblatt 94/43**

(84) Benannte Vertragsstaaten:
**AT BE CH DE DK ES FR GB GR IE IT LI LU MC NL PT SE**

(71) Anmelder: **Buchhold, Niels**
**Freiherr vom Stein Strasse 18**
**W-6361 Niddatal 1 (DE)**

(72) Erfinder: **Buchhold, Niels**
**Freiherr vom Stein Strasse 18**
**W-6361 Niddatal 1 (DE)**

(74) Vertreter: **Kruspig, Volkmar, Dipl.-Ing.**
**Patentanwälte**
**Meissner, Bolte & Partner**
**Postfach 86 06 24**
**D-81633 München (DE)**

(54) **Vorrichtung zur Steuerung peripherer Geräte mittels Zungenmotorik und Verfahren zur Auswertung von Steuersignalen.**

(57) Mit der Erfindung ist es möglich, schnell aufeinanderfolgende bzw. nacheinander vorliegende motorische Informationen mit hoher Sicherheit in elektrische Steuersignale umzuwandeln. Hierfür wird ein mit einem Zungenaufnahmestück (1) verbundener Stabmagnet (2) über einen multifunktionalen Kautschukring (4) zentriert und in definierter Lage in einem hohlzylindrischen Gehäuse (6) gelagert. In einem abgedichteten Bereich des hohlzylindrischen Gehäuses ist eine Vielzahl von Hall-Elementen (7) angeordnet, um eine analoge Hall-Spannung entsprechend der Bewegung des über das Zungenaufnahmestück mit Hilfe der Zungenmotorik bewegten Stabmagneten zu erfassen. Hierbei kann der Endzustand der Auslenkung des Stabmagneten und der Betrag und die Richtung der Bewegung unmittelbar und analog zur ausgeführten Betätigung durch die Zunge bestimmt werden. Durch eine minimale Anzahl von Konstruktionselementen im Mundstück können dessen Abmessungen verkleinert werden, so daß sich die Trage- bzw. Betätigungseigenschaften verbessern.

Figur 1

Die Erfindung betrifft eine Vorrichtung zur Steuerung pheripherer Geräte mittels Zungenmotorik und ein Verfahren zur Auswertung von Steuersignalen die aus einer derartigen Vorrichtung gewonnen werden nach dem Oberbegriff der Patentansprüche 1 und 7.

Es ist bereits vorgeschlagen worden, körperbehinderten Menschen, insbesondere Tetraplegikern eine Vorrichtung zur Verfügung zu stellen, mit deren Hilfe es unter Einsatz der Zungenmotorik möglich ist, periphere Geräte, z.B. Computer, Fernsehgeräte, Telefone und ähnliches anzusteuern. Hierdurch können die behinderten Menschen ohne Hilfe Dritter bestimmte Tätigkeiten selbständig durchführen.

So beschreibt die US-PS 4 728 812 eine Steuereinrichtung unter Nutzung der Zungenmotorik, wobei die Einrichtung zwei Tragplatten aufweist, mit deren Hilfe ein Fixieren durch die oberen und unteren Zahnreihen im Kiefer der betreffenden Personen möglich ist. Mit der Zunge bzw. der Zungenspitze werden dann entweder eine Kontaktgruppe oder ein Potentiometer betätigt, welches wiederum mit einer Auswerteelektronik in Verbindung steht. Nachteilig ist die relativ große Ausführung der Steuerungsvorrichtung, die sich im Gebrauch vor dem Mund befindet und die erforderliche extreme Auslenkung eines am Abgriff des erwähnten Potentiometers befestigten Betätigungsstiftes. Die beschriebene Steuereinrichtung kann aufgrund des gewählten Konstruktionsprinzips daher nicht für das Verarbeiten schnell aufeinanderfolgender Informationen eingesetzt werden.

Auch die den der CH-PS 660 956 beschriebene Steuervorrichtung für körperlich behinderte Personen, welche mit Hilfe eines einzigen Kontaktes verschiedene Steuermöglichkeiten erschließen soll, ist auf Grund des erforderlichen sequentiellen Verarbeitens der Information über einen einzigen Kontakt nicht geeignet schnell aufeinanderfolgende Informationen zu verarbeiten. Vielmehr muß sich der Bediener Schritt für Schritt zu einem jeweils gewünschten Menü vorarbeiten, um dann im Dialog mit dem jeweiligen Menü die entsprechende Steuerungsaufgabe zu lösen.

Aus der Deutschen Offenlegungsschrift 19 43 824 ist ein Verfahren zur Steuerung einer Vorrichtung zur Ortsveränderung durch einen Tetraplegiker sowie eine zugehörige Vorrichtung offenbart. Hierbei wird die Zungenmotorik derart genutzt, daß je nach einer beabsichtigten Fahrtrichtung eines mit der Steuervorrichtung gekoppelten Rollstuhls mit der Zunge das Schließen eines jeweils verschiedenen Elektroden zugeordneten Stromkreises erfolgt. Für jede Antriebsrichtung ist eine entsprechende Elektrodenanzahl vorzusehen, was zu einer räumlichen Vergrößerung der Steuereinrichtung führt. Zum Betätigen der Elektrode muß außerdem entweder die Elektrodenplatte zum Bediener hin oder umgekehrt bewegt werden , wodurch die Anwendung einer derartigen Vorrichtung in der Praxis erschwert wird.

Eine weitere bereits vorgeschlagene Technik besteht darin, daß der körperbehinderte Mensch mit dem Mund ein Rohr aufnimmt und durch Saugen oder Blasen in der Lage ist, zwei Informationen an eine entsprechende Auswerteeinrichtung zu liefern. Eine derartige Saug- Blastechnik weist aber die gleichen Nachteile hinsichtlich der schnellen Verarbeitung aufeinanderfolgender Informationen auf, wie sie beim unmittelbaren Betätigen von elektrischen Kontakten durch die Zunge vorhanden sind.

Die DE 41 00 402 A1 zeigt ein Behindertenmundstück zur Steuerung von Computern und anderen Hilfsgeräten durch Einsatz und Auswertung der Zungenmotorik.

Dieses dort gezeigte Mundstück ähnelt einem miniaturisierten Joystick, welcher derart verkleinert ist, daß er unmittelbar während der Benutzung im Mund befindlich sein kann. Eine Betätigung des Mundstückes erfolgt durch Einwirkung der Zungenkraft auf einen Käfig, welcher über eine Achse, die federbelastet ist, elektrische Kontakte schließen kann. Hierbei kann in quasi digitaler Weise das Schließen bestimmter Kontakte je nach der Art der Bewegung der Zunge erfolgen.

In jedem Fall ist es jedoch erforderlich, daß der mit der Zunge über eine Achse betätigte Klöppel in mechanischen und elektrischen Kontakt mit den zugehörigen Kontaktblättern bzw. dem Mittelkontakt kommt. Dies wiederum führt zu dem Problem, daß bei einem längeren Einsatz der im Mund befindlichen Betätigungsvorrichtung eine Kontaktoberflächenkorrosion auftritt, so daß eine sichere Kontaktgabe nicht mehr gewährleistet ist.

Ebenso besteht die Gefahr, daß ein Kontaktprellen zu einem fehlerhaften Auslesen führt.

Es besteht daher die Aufgabe der Erfindung, eine Vorrichtung zum Steuerung peripherer Geräte mittels der Zungenmotorik sowie ein Verfahren zur Steuersignal-Auswertung anzugeben, wobei die Vorrichtung direkt im Mund bzw. der Mundhöhle der Bedienperson befindlich sein kann und wobei eine analoge Signalausgabe bzw. Signalverarbeitung derart möglich ist, daß schnell aufeinander bzw. nacheinander vorliegende motorische Informationen mit hoher Sicherheit in entsprechende elektrische Steuersignale umgewandelt werden können.

Hierdurch soll die Verwendung der Vorrichtung zur Steuerung nicht nur für den Einsatz im persönlichen Bereich, z.B. zum Einschalten eines Fernsehgerätes oder zum Betätigen eines Telefons sondern auch im Bereich der Arbeitsumwelt gegeben sein und sich für den behinderten Menschen neue berufliche Tätigkeitsgebiete und Perspektiven im Sinne einer Rehabilitation ergeben.

Eine weitere Teilaufgabe der Erfindung besteht darin, daß die Vorrichtung zur Steuerung so konstruiert werden soll, daß ein intuitives schnelles Erlernen der Betätigung selbiger erreicht werden kann.

Die Lösung der Aufgabe der Erfindung erfolgt mit den Merkmalen der Patentansprüche 1 und 7, wobei die Unteransprüche vorteilhafte Ausgestaltungen und Weiterbildungen zeigen.

Der Grundgedanke der Erfindung besteht darin, eine langzeitstabile und betätigungsoptimale Vorrichtung zur Steuerung auf der Basis eines Mundstückes anzugeben, wobei ein mit einem Zungenaufnahmestück verbundener Stabmagnet über einen multifunktionalen Kautschukring zentriert und in definierter Lage in einem hohlzylindrischen Gehäuse gelagert ist und wo in einem abgedichteten Bereich des hohlzylindrischen Gehäuses eine Vielzahl von Hall-Elementen angeordnet ist, um eine analoge Hallspannung entsprechend der Bewegung des über das Zungenaufnahmestück mit Hilfe der Zungenmotorik bewegten Stabmageten zu erfassen.

Mit der erfindungsgemäßen Konstruktion gelingt es demnach nicht nur, den Endzustand der Auslenkung des Stabmagneten zu erfassen, sondern es kann der Betrag und die Richtung der Bewegung unmittelbar und analog zur ausgeführten Betätigung durch die Zunge bestimmt werden. Dadurch, daß keine elektromechanischen Kontaktelemente erforderlich sind und auf zusätzliche Federmittel zur Herstellung einer zentralen vorgegebenen Lage des Stabmagneten verzichtet werden kann, erhöht sich die Zuverlässigkeit der gesamten Vorrichtung.

Es liegt im Sinne der Erfindung, anstelle von Hall-Elementen ähnlich wirksame berührungslos arbeitende Sensoren einzusetzen. Durch die minimale Anzahl von Konstruktionselementen bzw. Bauelementen im Mundstück, können dessen Abmessungen weiter verkleinert werden, so daß sich die Trage- bzw. Betätigungseigenschaften verbessern.

Überraschenderweise ist es mit einer einzigen multifunktionalen Dichtscheibe, die eine vorgegebene Elastizität besitzt, möglich, eine Abdichtung gegen Speichel und einen fühlbaren Betätigungswiderstand bei gleichzeitigem Einhalten einer vorgegebenen Ausgangslage des Stabmagneten zu erreichen. Es wird also mit anderen Worten durch die spezielle Dichtscheibe eine horizontale und vertikale Zentrierung des Stabmagneten gesichert.

Im Ergebnis längerer Untersuchungen hat sich herausgestellt, daß bereits ein kurzes Antippen des Zungenaufnahmestückes und eine damit verbundene Elongation des Stabmagneten ausreicht, um ein definiertes Ausgangssignal für eine weitere Signalverarbeitung zu erhalten.

Auf Grund der besonderen Konstruktion des Mundstückes und der Art der Signalgewinnung durch Ableiten einer richtungs- und hubabhängigen Hallspannung können außerordentlich schnell aufeinander folgende zungenmotorische Impulse verarbeitet werden. Hierdurch ergeben sich völlig neue Anwendungsmöglichkeiten der Steuerungsvorrichtung, so daß diese auch zum Bewegen eines Fahrzeuges, z.B. eines Rollstuhles eingesetzt werden kann.

Durch das unmittelbare und schnelle Umsetzen der Zungenmotorik, d. h. der Steuerbefehle und die quasi parallele Signalverarbeitung können die für die Steuerung eines Fahrzeuges notwendigen kurzen Zyklen zwischen der visuellen Aufnahme, z.B. eines Hindernisses auf der Bewegungsbahn, dem Auslösen eines Befehles zum Umfahren dieses Hindernisses und der Ausgabe eines entsprechenden Fahrbefehls an eine Lenkvorrichtung realisiert werden.

In einer Ausführungsform der Erfindung kann die Vorrichtung zur Steuerung an einem Rollstuhl derart befestigt sein, daß der Tetraplegiker das Mundstück selbsttätig aufnehmen kann.

Neben der eigentlichen Steuerungsfunktion des Bewegens des Rollstuhles kann über ein einfaches Umschaltmenü das betätigen weiterer Geräte über einen am Rollstuhl befestigten Sender, der mit einem, z. B. zentral angeordneten Empfänger zusammenwirkt, realisiert werden. Zweckmäßigerweise erfolgt die kodierte Signalübertragung zwischen dem am Rollstuhl befindlichen Sender und dem Empfänger über eine Infrarotübertragungsstrecke oder durch hochfrequente elektromagnetische Strahlung.

In einer weiteren Ausführungsform der Erfindung ist der Rollstuhl mit einem speziellen optoelektronischen Modem ausgestattet, so daß der Benutzer auch ohne fremde Hilfe, z.B. eine mit einem baugleichen Modem ausgerüstete öffentliche Telefonzelle aufsuchen und Telefongespräche führen kann.

Das Verfahren zur Auswertung von Steuersignalen aus einer Vorrichtung zur Steuerung peripherer Geräte wird derart realisiert, daß die Signale der Hall-Elemente, die im hohlzylindrischen Mittelteil des Mundstückes in definierter Lage zueinander angeordnet sind, in parallelen Kanälen weiterverarbeitet werden. Jedem Hall-Element ist dabei im Übertragungskanal zunächst ein Verstärker nachgeordnet. Die aufgrund der Wirkungsweise der Hall-Elemente unstetigen Verläufe zwischen der Hall-Spannung U und dem Weg S, den der Stabmagnet, bezogen auf das betrachtete Hall-Element zurücklegt, können nicht unmittelbar zur analogen Steuerung eines Rollstuhles verwendet werden.

Aus diesem Grunde wird beim erfindungsgemäßen Verfahren eine Analog/Digitalwandlung des verstärkten Hall-Signals je Kanal vorgenommen und es erfolgt eine kanalbezogene Linearisierung durch Kennlienienapproximation mit Hilfe eines Mikrocontrollers.

Mit einem Datenübergabe-Baustein werden die linearisierten Signale entweder einer herkömmlichen PC-Steuerung zur Verfügung gestellt oder es erfolgt eine Digital/Analogwandlung zur unmittelbaren analogen Ansteuerung z. B. von Servomotoren einer Lenkvorrichtung zur Steuerung des Rollstuhles.

Die Erfindung soll nunmehr an Hand von Figuren und Ausführungsbeispielen näher beschrieben werden.

Hierbei zeigen

    Fig. 1    eine Längsschnitt Darstellung der Steuerungsvorrichtung;
    Fig. 2    eine prinzipielle Darstellung der Aufnahme der Steuerungsvorrichtung im Mund eines Tetraplegikers

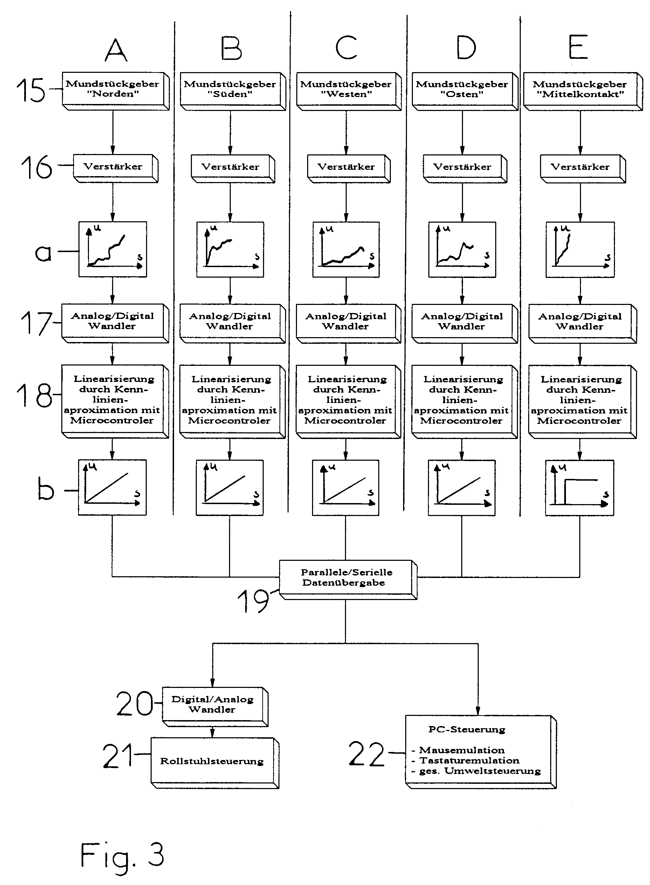

    Fig. 3    eine Blockschaltung zur Erläuterung des erfindungsgemäßen Signalausweteverfahrens.

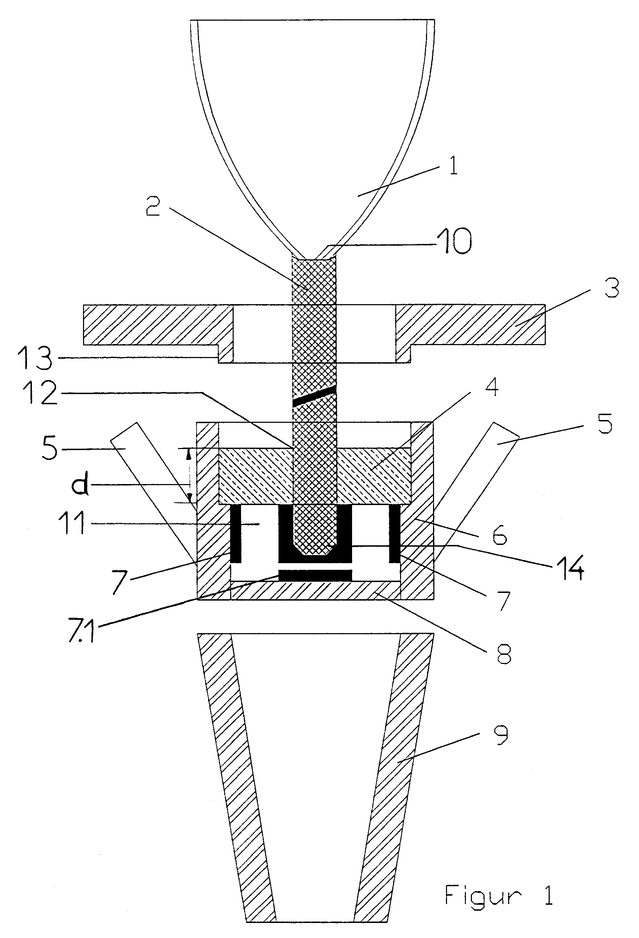

Die Steuerungsvorrichtung gemäß Fig. 1 bzw. das Mundstück besteht aus einem Zungenaufnahmeteil 1, welches im wesentlichen eine Trichterform aufweist. Am verjüngten unteren Ende des Zungenaufnahmeteiles 1 ist eine Bohrung 10 vorgesehen, die der Aufnahme eines Endes eines vorteilhafterweise zylindrischen oder einen anderen Querschnitt aufweisenden Stabmagneten 2 dient. Der Stabmagnet 2 mit Zungenaufnahmeteil 1 wird mit seinem anderen Ende durch einen speziellen elastischen Dichtring oder einer Dichtscheibe 4 fixiert.

Die Dichtscheibe bzw. der Dichtring 4 ist an einem oberen Ende in ein hohlzylindrisches Mittelteil 6 eingepaßt. Das hohlzylindrische Mittelteil 6 ist am unteren Ende mit einer Abschlußplatte 8 verschlossen. Die Abschlußplatte 8 und das hohlzylindrische Mittelteil 6 sind beispielsweise miteinander verklebt.

In dem von der Dichtscheibe 4 und der unteren Abschlußplatte 8 bzw. dem hohlzylindrischen Mittelteil 6 gebildeten Raum befinden sich eine Vielzahl von Hall-Elementen 7. Die Hall-Elemente 7 sind jeweils am Innenumfang des hohlzylindrischen Mittelteiles 6 sich gegenüberliegend, z. B. in Nord-Süd, Ost-West-Richtung angeordnet, wobei zusätzlich zentrisch an der Innenseite der unteren Abschlußplatte 8 ein weiteres Hall-Element 7.1 befindlich ist.

Der Dichtring 4 ist in seiner Dicke d in Abhängigkeit von den Elastizitätseigenschaften derart ausgebildet, daß er zum einen das Zungenaufnahmeteil 1 mit dem Stabmagneten 2 sicher führen kann und zum anderen eine ausreichende Abdichtung des Innenhohlraumes 11, in welchem sich die Hall-Elemente 7, 7.1 befinden, gewährleistet ist.

Durch die elastischen Eigenschaften des Dichtringes 4 und die Berücksichtigung eines vorgegebenen Verhältnisses zwischen dem Durchmesser des Stabmagneten 2 und der inneren Bohrung 12 der Dichtscheibe 4 sowie der Dicke d und dem Innendurchmesser des hohlzylindrischen Mittelteiles 6 wird der Bewegung des Stabmagneten 2 ein fühlbarer aber nicht zu großer Widerstand entgegengesetzt, so daß ein gefühlvolles und schnelles Betätigen des Zungenaufnahmeteiles 1 durch die Zunge des Probanden möglich wird.

Die untere Abschlußplatte 8 weist nicht gezeigte Durchführungsöffnungen zur Aufnahme der Anschlußleitungen der Hall-Elemente 7, 7.1 auf. Diese Durchführungsöffnungen werden beispielsweise mit einem Epoxidharz oder einem Polymermaterial abgedichtet.

Am oberen Ende des hohlzylindrischen Mittelteiles 6 ist eine obere Abschlußscheibe 3 mit Hilfe eines stufenförmigen Abschnittes bzw. eines Flansches 13 eingepaßt und gegebenfalls mit dem Mittelteil 6 verklebt. Der Außendurchmesser der Abschlußscheibe 3 ist dabei wesentlich größer als der Durchmesser des hohlzylindrischen Mittelteiles 6.

Ein abgewinkelter Kautschukring 5, der am Außenumfang des hohlzylindrischem Mittelteiles 6 befestigt ist, verbessert die Trage- und Betätigungseigenschaften der Steuerungsvorrichtung bzw. des erfindungsgemäßen Mundstückes.

Am unteren Ende des hohlzylindrischen Mittelteiles 6 bzw. der unteren Abschlußplatte 8 ist ein Gehäuseteil 9 vorgesehen, welches der Aufnahme und dem Führen der Verbindungskabel zu den Hall-Elementen 7 dient. Das Gehäuseteil 9 besitzt vorteilhafterweise eine konische Form und ist mit dem hohlzylindrischen Mittelteil 6 auf Stoß verklebt.

Die miniaturisierte Ausführung des Mundstückes wird deutlich, wenn man die Größenverhältnisse der einzelnen Bauteile betrachtet.

So besitzt beispielsweise das hohlzylindrische Mittelteil 6 einen Außendurchmesser von ca. 10 mm bei einer Höhe von 7 mm. Die Dicke der Dichtscheibe 4 liegt beispielsweise bei ca. 3 mm und der Durchmesser des Stabmagneten 2 beträgt ca. 2 mm.

Besonders vorteilhaft ist eine Dichtscheibe 4 aus Naturkautschukmaterial mit folgenden Relationen

Verhältnis der Innenbohrung des Stabmagneten zur Dicke d der Scheibe und zum Durchmesser der Scheibe von 1 : 1-1,5 : 3-4.

Zur Verbesserung der Auswertegenauigkeit bezogen auf die Bewegung des Zungenaufnahmeteiles 1 und des daran befestigten Stabmagneten 2, insbesondere hinsichtlich des zentrischen Hall-Elementes 7.1, ist der Stabmagnet 2 im unteren Bereich 14, der in den Hohlraum des hohlzylindrischen Mittelteiles 6 hineinreicht, angefast.

Die Ansteuerung und Auswertung der Hall-Elemente 7, 7.1 erfolgt derart, daß bei einem vorgegebenen Ruhezustand, der der zentrischen Lage des Stabmagnetens 2 entspricht, eine Offsetspannung eingestellt wird.

Je nach Bewegung, nach Intensität und Richtung der Auslenkung des Stabmagneten 2 erfolgt eine Veränderung der Hallspannungen der umgebenden Hall-Elemente 7, 7.1. Durch eine Auswertung und einen Vergleich der sich verändernden Spannungen und gegebenenfalls einen Zeitbezug können auch geringe, schnell aufeinanderfolgende Elongationen des Stabmagneten 2 sicher erfaßt werden.

Durch die gewählte Konstruktion können auch quasi überlagerte Bewegungen, z.B. eine seitliche Bewegung mit einer axialen Bewegung, sicher erfaßt und definierten Befehlen zugeordnet werden.

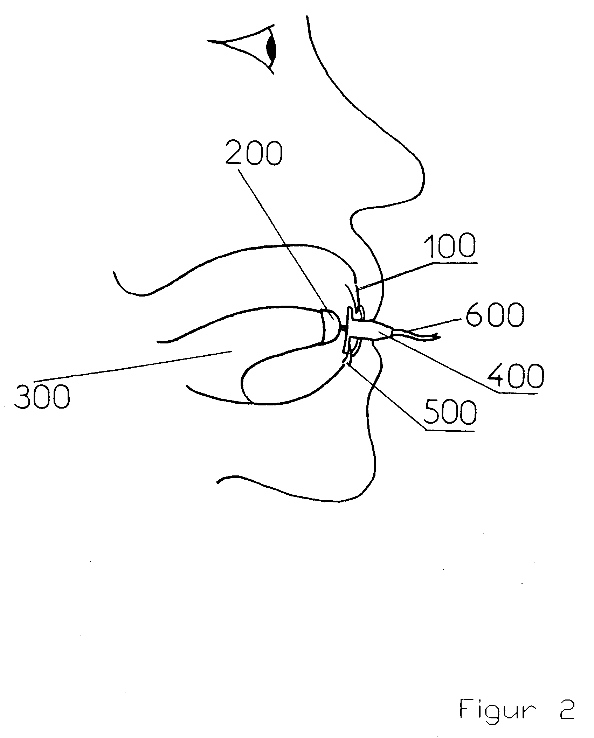

Mit Hilfe der Fig. 2 soll das Tragen der Steuerungsvorrichtung bzw. des Mundstückes 400 im Mund des Probanden deutlich gemacht werden.

Mit der Zunge 300 erfolgt eine innige Berührung durch Ausfüllen des trichterförmigen Hohlraumes des Zungenaufnahmeteiles 200. Die Bewegung der Zunge 300 wird über das Zungenaufnahmeteil 200 auf den hier nicht gezeigten Stabmagneten übertragen und durch die Hall-Elemente erfaßt.

Das Mundstück 400 wird zwischen oberer 100 und unterer 500 Zahnreihe eingeklemmt und dadurch fixiert.

Durch die erwähnten geringen Abmessungen des Mundstückes und die flexible Verbindung in Form weniger elektrischer Leiter 600 zur Auswertereinrichtung bleibt der Bewegungsspielraum des Kopfes des Probanden im wesentlichen unbeeinträchtigt.

Dadurch, daß das Mundstück größtenteils in der Mundhöhle befindlich ist, können schnell und unauffällig Signale ausgelöst werden. Wenn das Mundstück an einer Art Teleskopstab oder einem bewegliche Schwanenhalsarm, zum Beispiel an einem Rollstuhl befestigt ist, kann es der behinderte Mensch leicht und wiederholt aufnehmen und abgeben.

Die wesentlichen Bestandteile des in den Fig. 1 und 2 gezeigten Mundstückes bestehen z.B. aus einem verträglichem Kunststoff, wie z. B. Paladur.

Mit Hilfe der Fig., welche ein Blockschaltbild der Signalverarbeitungsbaugruppen darstellt, soll das Verfahren zur Auswertung der Steuersignale erläutert werden.

Beim gezeigten Beispiel ist eine fünfkanalige Anordnung vorgesehen, wobei jedes an einem definiertem Ort im Mundstückgeber 15 befindliche Hall-Element einem Kanal A - E zugeordnet ist.

Die Ausgangssignale der Hall-Elemente werden über nachgeschaltete Verstärker 16 auf einen Analog/Digitalwandler 17 geführt.

Mit den im Blockschaltbild gezeigten Kurvenverläufen a) und b) soll die dem betreffenden Weg bzw. der Elongation S des Stabmagneten zugeordnete Spannung U symbolisch dargestellt werden.

Da, wie aus a) ersichtlich der Spannungsverlauf noch eine Vielzahl von Unstetigkeiten aufweist, ist eine unmittelbare Auswertung nicht oder nur unzureichend möglich. Auf Grund dessen wird mit den bereits erwähnten Analog/Digitalwandlern 17 eine Digitalisierung und in Linearisierungsbausteinen mit Hilfe eines Mikrorechners eine Linearisierung der Spannungsverläufe a) durch Approximation vorgenommen.

Die am Ausgang der Linearisierungsbausteine anliegenden Signale (b) werden auf eine parallel/serielle Datenübergabebaugruppe 19 geführt. Das am Ausgang der Baugruppe 19 anliegende Signal kann entweder über einen Digital/Analogwandler 20 in ein analoges Signal umgesetzt werden, welches z. B. unmittelbar für ein analoge Rollstuhlsteuerung verwendbar ist, oder direkt zu einer PC Steuerung 22 zum Betreiben peripherer Geräte führen.

Mittels der Erfindung ist es zusammenfassend möglich, einem behinderten Menschen nicht nur eine wirkliche Lebenshilfe zu bieten, deren Handhabung leicht und quasi intuitiv erlernbar ist, sondern es ist eine wirkliche Rehabilitierung unter Ausnutzung aller vorhandener Fähigkeiten gegeben. Durch die Möglichkeit des schnell aufeinanderfolgenden Aussendens von Informationen auf der Basis der Zungenmotorik können nicht nur einfache Schaltvorgänge sondern auch kompliziertere Steuerungsaufgaben, z.B. Betätigen einer CAD-Workstation gelöst werden.

Der Einsatz der erfindungsgemäßen Vorrichtung und des Signalauswerteverfahrens gestattet daher ein kreatives Arbeiten an bzw. mit einem Computer.

## Patentansprüche

1.  Vorrichtung zur Steuerung peripherer Geräte mittes Zungenmotorik, umfassend:
    ein im wesentlichen in der Mundhöhle befindliches Mundstück mit einem Zungenaufnahmeteil, welches über eine Achse eine Bewegung der Zunge in ein hohlzylindrisches, bodenseitig geschlossenes Mittelteil zur Steuersignalgabe überträgt
    **gekennzeichnet durch**
    -   einen in das hohlzylindrische Mittelteil (6) eingepaßten elastischen Dichtring bzw. Dichtscheibe (4), wobei die Achse aus einem Stabmagneten (2) besteht, welcher in einer Mittelbohrung (12) des Dichtringes bzw. der Dichtscheibe (4) straff geführt und zentriert ist und

- im hohlzylindrischen Mittelteil (6) eine Vielzahl von Hall-Elementen (7, 7.1) derart angeordnet ist, daß die über die Achse bzw. den Stabmagneten (2) übertragene Zungenmotorik nach Betrag und Richtung analog erfaßbar ist.

2. Vorrichtung nach Anspruch 1,
   **dadurch gekennzeichnet**,
   daß die Hall-Elemente (7) am Innenumfang des hohlzylindrischen Mittelteiles (6) sich jeweils gegenüberliegend und mindestens ein Hall-Element (7.1) zentrisch an der Innenseite einer unteren Abschlußplatte (8) angeordnet sind.

3. Vorrichtung nach Anspruch 1 und 2,
   **dadurch gekennzeichnet**,
   daß der Stabmagnet (2) an seinem in das hohlzylindrische Mittelteil (6) hineinragenden Ende angefast ist.

4. Vorrichtung nach Anspruch 1 bis 3,
   **dadurch gekennzeichnet**,
   daß der elastische Dichtring bzw. die Dichtscheibe (4) im wesentlichen ein Verhältnis von Durchmesser der Mittelbohrung zur Scheibendicke und zum Gesamtdurchmesser von

   1 : 1 - 1,5 : 3 - 4

   aufweist.

5. Vorrichtung nach Anspruch 1,
   **dadurch gekennzeichnet**,
   daß am oberen Abschluß des hohlzylindrischen Mittelteiles (6) eine Abschlußscheibe (3) mit Hilfe eines stufenförmigen Abschnittes bzw. eines Flansches (13) befestigt ist, wobei die obere Abschlußscheibe (3), bezogen auf das hohlzylindrische Mittelteil (6) überstehend ausgebildet ist.

6. Vorrichtung nach Anspruch 1,
   **dadurch gekennzeichnet**,
   daß am Außenumfang des hohlzylindrischen Mittelteiles (6) ein abgewinkelter Kautschukring (5) befestigt ist.

7. Verfahren zur Auswertung von Steuersignalen aus einer Vorrichtung gemäß den Ansprüchen 1 bis 6,
   **dadurch gekennzeichnet**,
   daß jedem Hall-Element ein Signalverarbeitungskanal zugeordnet ist und die zunächst analog/digital gewandelten wegabhängigen Spannungssignale der Hall-Elemente einer Kennlinienapproximation zur Linearisierung unterzogen werden, anschließend wahlweise eine Digital/Analogwandlung der linearisierten Spannungssignale erfolgt und wobei die wahlweise erhaltenen Analogsignale zur unmittelbaren Steuerung z. B. eines Antriebes für einen Rollstuhl verwendet werden.

8. Verfahren nach Anspruch 7,
   **dadurch gekennzeichnet**,
   daß die Linearisierung der Kennlinien mittels eines Mikrorechners durchgeführt wird.

9. Verfahren nach Anspruch 7 und 8,
   **dadurch gekennzeichnet**,
   daß die Ausgangssignale der Signalverarbeitungskanäle nach der Kennlinienlinearisierung in einer parallel/seriellen Datenübergabebaugruppe zusammengefaßt und entweder einer PC-Steuerung zur Beeinflußung peripherer Geräte und/oder einer Digital/Analogwandlung zur Ableitung von analogen Steuersignalen zugeführt werden.

Figur 1

Figur 2

|  | A | B | C | D | E |
|---|---|---|---|---|---|
| 15 | Mundstückgeber "Norden" | Mundstückgeber "Süden" | Mundstückgeber "Westen" | Mundstückgeber "Osten" | Mundstückgeber "Mittelkontakt" |
| 16 | Verstärker | Verstärker | Verstärker | Verstärker | Verstärker |
| a | | | | | |
| 17 | Analog/Digital Wandler | Analog/Digital Wandler | Analog/Digital Wandler | Analog/Digital Wandler | Analog/Digital Wandler |
| 18 | Linearisierung durch Kenn-linien-aproximation mit Microcontroler | Linearisierung durch Kenn-linien-aproximation mit Microcontroler | Linearisierung durch Kenn-linien-aproximation mit Microcontroler | Linearisierung durch Kenn-linien-aproximation mit Microcontroler | Linearisierung durch Kenn-linien-aproximation mit Microcontroler |
| b | | | | | |

19 — Parallele/Serielle Datenübergabe

20 — Digital/Analog Wandler

21 — Rollstuhlsteuerung

22 — PC-Steuerung
- Mausemulation
- Tastaturemulation
- ges. Umweltsteuerung

Fig. 3

9

Europäisches
Patentamt

EUROPÄISCHER RECHERCHENBERICHT

Nummer der Anmeldung

EP    93 10 6409

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | DE-U-9 100 181 (BUCHHOLD)<br>* das ganze Dokument *<br>--- | 1-5 | A61F4/00<br>H01H3/14<br>G06F3/00 |
| Y | EP-A-0 501 906 (HARDI INTERNATIONAL A/S)<br>* Spalte 2, Zeile 26 - Spalte 3, Zeile 8; Abbildungen 1-3 *<br>--- | 1-5 | |
| Y,D | DE-A-4 100 402 (BUCHHOLD)<br>* Ansprüche 1-3; Abbildungen *<br>--- | 7-9 | |
| Y | EP-A-0 399 484 (FERNSTEUERGERÄTE KURT OELSCH KOMMANDITGESELLSCHAFT)<br>* Zusammenfassung; Abbildungen 1-3 *<br>--- | 7-9 | |
| A | US-A-4 458 226 (MATAHARI INT. CORP.)<br>* Zusammenfassung *<br>--- | 1 | |
| A | US-A-4 462 015 (NETZER)<br>* Zusammenfassung *<br>--- | 1 | |
| A,D | US-A-4 728 812 (SHERIFF ET AL.)<br><br>----- | | RECHERCHIERTE SACHGEBIETE (Int. Cl.5 )<br><br>A61F<br>G06F<br>H01H<br>G06K |

Der vorliegende Recherchenbericht wurde für alle Patentansprüche erstellt

| Recherchenort | Abschlußdatum der Recherche | Prüfer |
|---|---|---|
| DEN HAAG | 02 AUGUST 1993 | SANCHEZ Y SANCHEZ J. |